# EUROPEAN PATENT APPLICATION

(11) **EP 1 266 672 A1**
(43) Date of publication of application: **18.12.2002**
(21) Application number: 01912492.4
(22) Date of filing: 19.03.2001
(51) Int. Cl.: A61N 1/04, A61N 1/32

(54) **PULSE HEALTH DEVICE**

(30) Priority: 24.03.2000 JP 2000084980
(71) Applicant: Yaman Ltd., Tokyo 135-0045 (JP)
(72) Inventor: Yamazaki, Iwao, C/O Yaman Ltd., Tokyo 135-0045 (JP); Izawa, Yoshihiro, C/O Yaman Ltd., Tokyo 135-0045 (JP)
(74) Representative: Newstead, Michael John
(86) International application number: JP0102174
(87) International publication number: WO01070333

(57) **Abstract**

Disclosed is a health-care pulse stimulator which is capable of feeding the abdomen with pulse current while the abdomen is reshaped by pushing up the lower part of the abdomen.

A broad belt of sheet-like insulating material S has electrode planes E1 to E3 arranged symmetrically with respect to the center line and fixed to the rear side of the broad belt. These electrode planes are so placed that they may be applied to the opposite sides, front and lower part of the abdomen of a living body. The broad belt has electrically conductive snaps H fixed to its front side for connecting the electrode planes to a pulse source. The broad belt has two cuts "a" extending inward from its opposite ends, thus forming upper, longer and lower, shorter sections A1 and A2. The upper, longer section A1 has a loop tape applied wholly to its front side, and it has a hook strip F applied to one end of the rear side. Thus, the upper, longer section can be wound tightly around the waist irrespective of the waist size.

The lower, shorter section A2 has hook strips F attached to the opposite ends of the rear side. The lower, shorter section A2 is applied to the lower part of the abdomen to push up it by pulling up the opposite ends of the lower section A2 and by allowing the hook strips F to be caught by the loop tape of the front side of the upper, longer section A1.

## Description

### Technical Field

The present invention relates to a health-care pulse stimulator whose electrodes are adapted to be put in contact with one's abdomen to be supplied with electric current pulses, thereby electrically stimulating the abdomen of a living body to give a nice shape to the living body.

### Background Art

The body fat takes a certain important role in the living body, but an excessive deposition of body fat is supposed to induce common diseases that elderly people are apt to get (hereinafter called "adult's diseases").

The overweight causes the blood pressure to rise undesirably high for circulating the blood in the living body.

The fat can be composed of carbohydrate and lipids, and an excessive blood sugar is apt to induce hyperlipemia, which can cause arteriosclerosis when combined with other certain factors.

The arteriosclerosis is apt to lead to coronary artery diseases such as the angina or the cardiac infarction in one's heart, and to the commonly called "cerebral apoplexy" such as the cerebral hemorrhage and the cerebral infaretion in one's brain.

An excessive deposition of fat in the liver results in the fatty liver, and an excessive amount of cholesterol, which is the major element of bile, is apt to produce a cholelithiasis. An excessive intake of meat and alcohol results in corpulence, and sometimes the gout is caused.

As may be understood from the above, the corpulence is closely related with adult's diseases. Therefore, it is important from the point of health-care view to prevent elderly persons from becoming fat.

The corpulence may be defined as having an excessive amount of body fat, and the corpulence which is closely related with adult's diseases is the visceral fat type corpulence or the corpulence of the upper half of the living body, which is called the "apple-shaped corpulence rather than the subcutaneous fat type corpulence or the corpulence of the lower half of the living body, which is called the "pear-shaped corpulence".

The visceral fat type corpulence is caused by deposition of fat around the abdomen, and therefore, removal of excessive amount of fat from the abdomen is effective in preventing adult's diseases, thereby keeping elderly persons good in health.

As is well known, bio-electric current flows ceaselessly in the living body to cause both effects of cell activation and muscular contraction.

The health-care pulse stimulator is used by wearing around the waist with its electrodes applied to selected areas of the living body, and by supplying the electrodes with electric pulse current for stimulating the living body. Like the bio-electric current the stimulation activates cells to expedite the metabolism and induce the muscular contraction. Thus, the extra amount of fat is made to burn.

The health-care pulse stimulator has the effect of reducing extra amount of fat by stimulation like massage or exercise. Fat can be effectively removed by rubbing the abdomen upwards.

Similarly fat can be removed effectively by making the electric pulse current flow while keeping the abdomen raised upward in tight condition.

In view of this a health-care pulse stimulator is so designed according to the present invention that electric pulse current may be made to flow while keeping the abdomen raised upward in tight condition.

### Disclosure of Invention

To attain such an objective a health-care pulse stimulator is constructed according to the present invention as follows:
a health-care pulse stimulator as claimed in claim 1 comprises: a broad belt of sheet-like insulating material having a plurality of electrode planes of soft material fixed to its rear side, the electrode planes being placed to be applied to selected areas of the abdomen when the broad belt is worn around the waist; and a pulse current source to be connected to the electrode planes, the broad belt being composed of upper and lower sections integrally connected at their intermediate portions, each section having fasteners fixed thereto thereby enabling the upper section to wear tightly around the waist, and the lower section to wear tightly around the lower part of the abdomen to raise the same upward;
a health-care pulse stimulator as claimed in claim 2 has the electrode planes so arranged to be applied to the opposite sides, front and lower part of the abdomen when the broad belt is worn around the waist;
a health-care pulse stimulator as claimed in claim 3 has a broad belt comprising longer, upper and shorter, lower sections integrally connected at their intermediate portions; and
a health-care pulse stimulator as claimed in claim 4 has terminals aligned in line and fixed to the broad belt, the electrode planes being adapted to be connected to the pulse source via the terminals.

### Brief Description of Drawings

Fig.1 is a front view of an electrode applier of a health-care pulse stimulator according to one embodiment of the present invention;
Fig.2 is a block diagram of a pulse source in the health-care pulse stimulator; and
Fig.3 illustrates how the electrode applier is used.

### Best Mode for Carrying Out the Invention

Referring to the accompanying drawings a health-care pulse stimulator according to one embodiment of the present invention is described below.

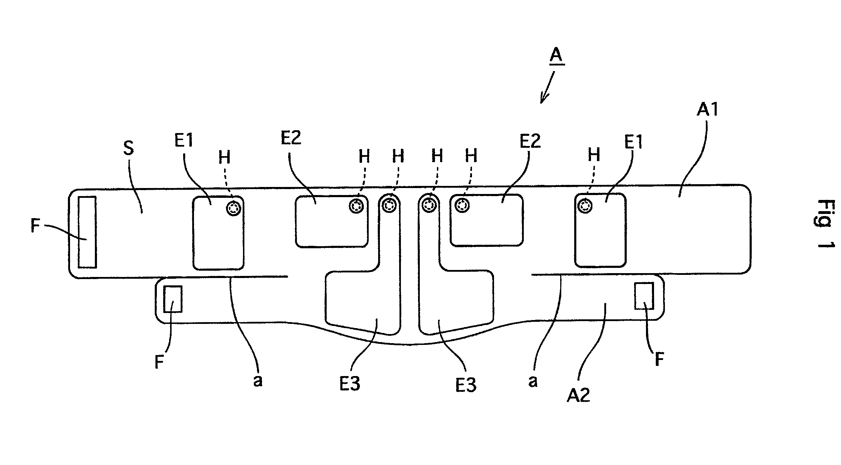

Fig.1 is a front view of the electrode applier A. It comprises a broad belt of sheet-like insulating material S having six electrode planes E1 to E3 fixed on its rear side. These electrode planes are arranged symmetrically on either side of the center line, three electrodes on either side being so arranged that they may be put in contact with the corresponding side of the abdomen when the broad belt is worn around the waist. Also, the broad belt has electrically conductive ball side (or socket side) snaps H fixed to its front side, the snaps H being aligned in line on the upper edges of the electrode planes E1 to E3.

An associated connector strip has electrically conductive socket side (or ball side) snaps fixed thereto, these snaps being arranged to be mated with the counter snaps of the broad belt.

The electrode planes E1 to E3 are so arranged that they may be applied to the upper part, front and lower part on either side of the abdomen when the broad belt is worn around the waist.

The broad belt of sheet-like insulating material comprises longer, upper and shorter, lower sections A1 and A2 integrally connected at their intermediate portion. The longer, upper and shorter, lower sections A1 and A2 are separated by the opposite cuts "a" in the broad band.

The longer, upper section A1 has a loop tape attached to the whole area of the front side and a hook tape F attached to one end of the rear side, thereby permitting the longer upper section to be wound and bound tightly around the waist irrespective of the waist size.

The shorter, lower section A2 has two hook tapes F attached to its opposite ends on the rear side, thus permitting the shorter, lower section A2 to embrace and raise the lower part of the abdomen with the opposite hook tapes F engaged with the loop tape of the front side of the longer, upper section.

Thus, the lower part of the abdomen is pulled up by the shorter, lower section A2 to give a good shape to the abdomen.

As described above, the loop tape is attached to the whole area of the front side of the longer, upper section A1, and therefore, the electrode applier A can be easily adjusted in length to fit the waist size by allowing the hook tapes F of the longer, upper and shorter, lower sections A1 and A2 to be mated with selected areas of the loop tape of the front side of the longer, upper section A1.

The loop tape may be attached to a selected area of the front side of the longer, upper section A1, which selected area is large enough to permit the hook tapes of the longer, upper and shorter, lower sections A1 and A2 to mate with selected portions of the loop tape in tight binding condition.

The electrode applier can be provided by stamping a piece of flexible cloth into a desired broad belt shape.

The stamping of texture material saves laborious sewing work, and accordingly the manufacturing cost can be reduced.

The electrode applier may be coated with medical adhesive agent on its rear side so that it may be applied directly to the living body in tight condition.

Each electrode plane may be provided by coating a piece of pliable urethane resin with carbon ink, and the so formed electrode plane may be attached to the rear side of a broad belt of insulating material S by sewing or welding the electrode plane with its coating up.

Otherwise, electrode planes may be printed on the broad belt S with carbon ink repeatedly in overlying lamination.

Thus, the electrode planes are formed to be coextensive with the broad belt, providing no irregular surface.

The electrode planes E1 to E3 may be lined with air-filled or sponge pads to make them closely fit the living body.

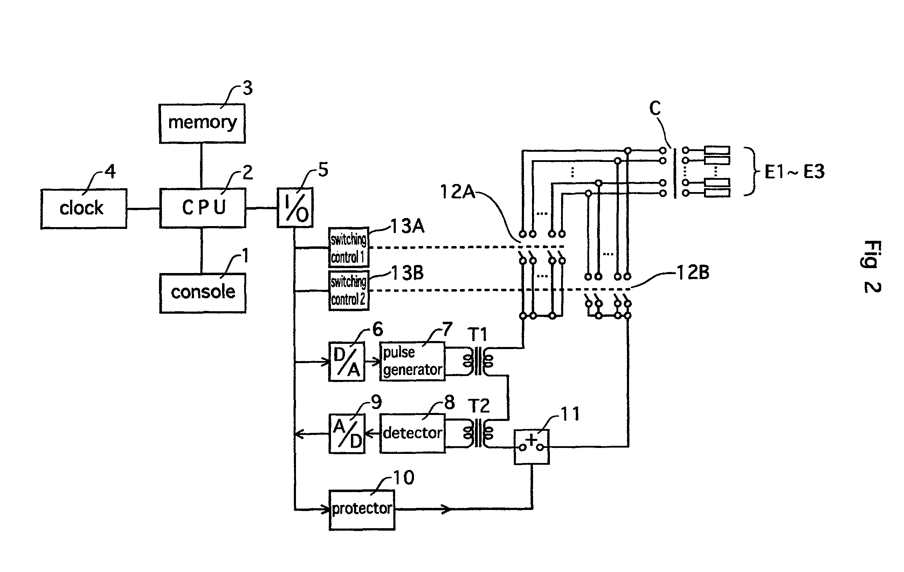

Fig.2 shows a block diagram of the pulse source used in the health-care pulse stimulator.

As shown, the pulse source comprises a console 1, a CPU 2 connected to the console 1, a memory 3 associated with the CPU 2, a reference clock 4 connected to the CPU 2, an I/O interface 5 connected to the CPU 2, a D/A converter 6 connected to the I/O interface 5, a pulse generator 7 connected to the D/A converter 6, a first transformer T1 whose primary winding is connected to the pulse generator T1, a second transformer T2 parallel-connected to the first transformer T1, a current detector 8 connected to the primary winding of the second transformer T2, an A/D converter 9 connected between the current detector 8 and the I/O interface 5, a protector circuit 10 connected to the I/O interface 5, a cutoff switch 11 connected to the protector circuit 10, switching circuits 12A and 12B whose first ends are connected together to the opposite ends of the series-connected secondary windings of the transformers T1 and T2 via the cutoff switch 11, the second ends of these switching circuits 12A and 12B being connected to the electrodes E1 to E3 via associated connectors C, and switching controls 13A and 13B connected between the I/O interface 5 and the switching circuits 12A and 12B. In operation the CPU 2 responds to the operation of the console 1 for retrieving pieces of information representing the kind of treatment and the current-feeding pattern from the memory 3, and a series of clock pulses from the reference clock 4 are divided to provide trigger signals according to the retrieved pieces of information. The trigger signals are directed to the pulse generator 7 via the I/O interface 5 and the D/A converter 6 to provide pulse signals of controlled duration and recurrence, which pulse signals are directed to the primary winding of the transformer T1.

The current detector 8 is connected across the secondary winding of the transformer T2, watching whether excessive quantity of electric current flows or not.

The electric current detected by the current detector 8 is directed to the CPU 2 via the A/D converter 9 and the I/O interface 5, and when the electric current thus detected increases beyond the threshold, the protector circuit 10 actuates the cutoff switch 11 to open the circuit.

The switching circuits 12A and 12B are optically connected to the switching controls 13A and 13B respectively via photo-couplers to be responsive to the signals appearing at the output terminals of the switching controls 13A and 13B for turning the switching circuits 12A and 12B on and off.

Thus, required pulse current is directed to each of the electrode planes E1 to E3 according to the selected kind of treatment and current feeding pattern.

As for the kinds of treatment to be effected "Toning" has the massaging effect of stimulating the depth of the living body, moving skeletal muscles and expediting the circulation of blood by using pulses at a reduced recurrence of 5 to 10 Hz, and "Drainage" has the massaging effect of stimulating the surface of the living body, moving upper layer muscles, helping the lymphatic circulation and removing edemas by using pulses at an increased recurrence of 20 to 100 Hz.

These treatments have their extra modes by changing cyclically the direction in which pulse current flows, thereby stimulating the living body cyclically in different fashion.

Also, "Toning" and "Drainage" still have their different versions. In one version the electrodes are supplied with pulse current in time-divisional fashion, and in the other version the electrodes are supplied with pulse current in simultaneous and recurrent fashion.

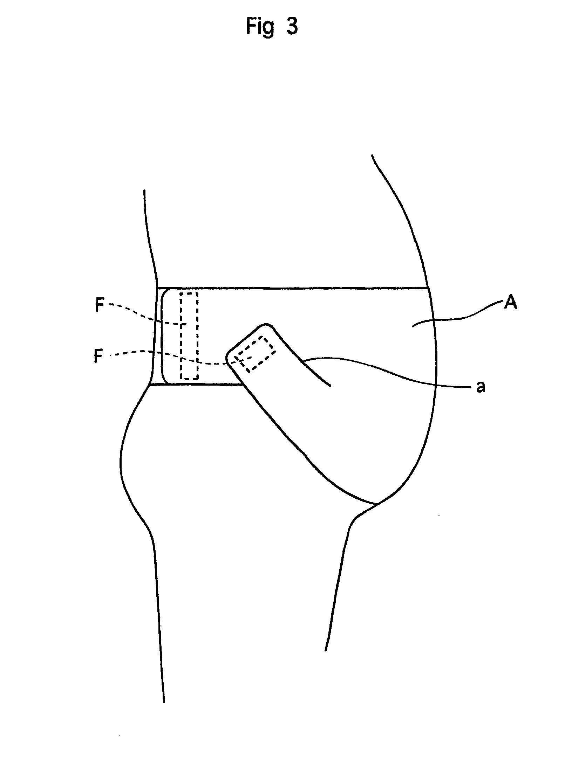

Referring to Fig.3, the electrode applier A is applied to the living body with the upper section A1 of the broad belt wound tightly around the waist. Specifically the upper section A1 binds the waist tightly by applying the hook tape F of the free end of the rear side of the upper section A1 to a selected portion of the front side of the loop tape of the upper section A1 to be caught thereby.

The electrode applier A may be moved around the waist until the electrodes E1 to E3 have come to the desired areas of the living body.

The hook tape F of the free end of the upper section A1 may be adjustably displaced to control the tightness with which the upper section A1 is wound around the waist.

Then, the lower section A2 is raised to pull up the lower part of the abdomen, and the lower section A2 is fastened by allowing the opposite hook tapes F to be caught by the loop tape of the upper section A1.

Thus, a desired treatment can be effected by selecting the kind of treatment and the current feeding pattern.

### Industrial Applicability

As described above, the health-care pulse stimulator comprises a broad belt of sheet-like insulating material having electrode planes attached to its rear side and fastener means attached to the electrode planes. In use, the broad belt is worn around the waist by winding its upper, longer section tightly around the waist, and by applying its lower, shorter section to the lower part of the abdomen to pull up the same. The opposite free ends of the lower, shorter section are adjustably fastened to the upper, longer section.

Thus, the abdomen is shaped by pulling up its lower part with the lower, shorter section of the broad belt and by stimulating the abdomen with pulse current. Partly because of the pulling up of the lower part of the abdomen and partly because of cyclic stimulation of the abdomen the reshaping effect is multiplied to give a pleasing shape to the living body.

## Claims

1. A health-care pulse stimulator comprising: a broad belt of sheet-like insulating material having a plurality of electrode planes of soft material fixed to its rear side, the electrode planes being placed to be applied to selected areas of the abdomen when the broad belt is worn around the waist; and a pulse current source to be connected to the electrode planes, the broad belt being composed of upper and lower sections integrally connected at their intermediate portions, each section having fasteners fixed thereto thereby enabling the upper section to wear tightly around the waist, and the lower section to wear tightly around the lower part of the abdomen to raise the same upward.

2. A health-care pulse stimulator according to claim 1 wherein the electrode planes are so arranged to be applied to the opposite sides, front and lower part of the abdomen when the broad belt is worn around the waist.

3. A health-care pulse stimulator according to claim 1 wherein the broad belt comprises longer, upper and shorter, lower sections integrally connected at their intermediate portions.

4. A health-care pulse stimulator according to claim 1 wherein it has terminals aligned in line and fixed to the broad belt, the electrode planes being adapted to be connected to the pulse source via the terminals.
